## Europäisches Patentamt
## European Patent Office
## Office européen des brevets

(19)

(11) Publication number: **0 036 662**
**B1**

(12)

# EUROPEAN PATENT SPECIFICATION

(45) Date of publication of patent specification: **16.11.83**

(51) Int. Cl.³: **C 12 N 11/08, C 12 P 19/24**

(21) Application number: **81102183.1**

(22) Date of filing: **23.03.81**

(54) An immobilized glucose isomerase system and a process for the isomerization of glucose using said system.

(30) Priority: **24.03.80 US 132728**

(43) Date of publication of application:
**30.09.81 Bulletin 81/39**

(45) Publication of the grant of the patent:
**16.11.83 Bulletin 83/46**

(84) Designated Contracting States:
**DE IT**

(56) References cited:
EP - A - 0 014 866
GB - A - 2 022 096
US - A - 3 960 663

DIE STÄRKE, vol. 27, no. 9, 1975 WEINHEIM
(DE) Y. YOKOTE et al.: "Production of high
fructose syrup gy G.I. immobilized on phenol-
formaldehyde resin", pages 302-306

PATENTS ABSTRACTS OF JAPAN, vol. 2, no.
122, October 13, 1978, page 2403 C 78

(73) Proprietor: **CPC INTERNATIONAL INC.**
**International Plaza P.O. Box 8000**
**Englewood Cliffs New Jersey 07632 (US)**

(72) Inventor: **Walon, Raoul G.P.**
**25, Avenue d'Hulderghem rez-de-chaussée**
**B-1020 Brussels (BE)**
Inventor: **Stouffs, Robert H.M.**
**336 Boulevard de Souverain**
**B- 1160 Brussels (BE)**

(74) Representative: **Lederer, Franz, Dr. et al,**
**Patentanwälte Dr. Franz Lederer Dipl.-Ing. Reiner**
**F. Meyer-Roxlau Lucile-Grahn-Strasse 22**
**D-8000 München 80 (DE)**

Courier Press, Leamington Spa, England.

# 0 036 662

An immobilized glucose isomerase system and a process for the isomerization of glucose
using said system

This invention relates to an immobilized glucose isomerase system and to a method of isomerizing glucose.

Glucose isomerase is the general name for the enzyme class which converts glucose (dextrose) into levulose (fructose). Since glucose isomerase was discovered as a product of microorganisms, the industrial production of levulose-bearing syrup from glucose on an industrial scale has become a major commercial process.

While levulose-bearing syrup can be prepared in a batch system using cells containing glucose isomerase, immobilized enzymes are preferred because they facilitate continuous as opposed to batch processing. Thus, methods of immobilizing glucose isomerase on various supports have been proposed and described in the prior art.

For example, immobilization of glucose isomerase on certain inorganic carriers is described in U.S. Patent Nos. 3,556,945 and 3,992,329.

The use of cellulose derivatives and synthetic resins as supports for glucose isomerase has also been described in the literature. Thus, U.S. Patent No. 3,909,354 describes the conversion of glucose to levulose using glucose isomerase bound to either an anion exchange cellulose or a synthetic anion exchange resin. U.S. Patent No. 3,960,663 discloses dextrose isomerase immobilized on porous or macroreticulated strongly basic anion exchange resin. In "Die Stärke" 1975, 302 glucose isomerase immobilized on phenol-formaldehyde resin is described. U.S. Patent No. 4,078,970 discloses glucose isomerase adsorbed and bound on an anion exchange resin having a porosity of more than 4.5% measured according to an aqueous dextrose solution method and an ion exchange capacity of more than 0.035 meq/ml resin measured according to a polyanion salt decomposition method. The resins are prepared by suspension polymerization of styrene, divinyl benzene, polystyrene and toluene in water using benzoyl peroxide as a catalyst, followed by chloromethylation and introduction of anion exchange groups. Japanese Specification No. NS80160/74 describes the immobilization of glucose isomerase on specific polyphenol resins, while Japanese Patent Specification No. S48—56770 describes the use of triazine derivatives to bind enzymes to polystyrene, polyphenol, alkylene, polyamine and aliphatic amine ion exchange resins. In Belgian Patent No. 862,765, a macroporous enzyme support having no ionic characteristics is described.

While many of the previously described supports for glucose isomerase do function to produce levulose-bearing syrups, they possess certain disadvantages. For example, some carriers result in poor enzyme activity, while other carriers require low throughput. Furthermore, many of these carriers are expensive and some must be regenerated and reused several times, adding to manufacturing costs.

Notwithstanding the plethora of enzyme supports known in the prior art, it has been discovered that a phenolic resin having a specific porosity profile has surprising and unexpected properties when used as a support for a specific glucose isomerase.

More in detail, this invention is directed to an immobilized glucose isomerase system that includes a glucose isomerase derived from *Streptomyces olivochromogenes* ATCC Nos. 21,114; 21,713; 21,714 or 21,715. Deposited strains ATCC Nos. 21,713; 21,714 and 21,715 are mutant strains of *Streptomyces olivochromogenes* ATCC No. 21,114. The glucose isomerase can also be derived from mutant strains of the ATCC. No. 21,715 microorganism. A commercially available glucose isomerase that is derived from the ATCC No. 21,715 microorganism or mutant strains thereof is sold under the name G—993 by CPC International Inc., International Plaza, Englewood Cliffs, New Jersey, U.S.A. The glucose isomerase is immobilized on a porous phenolic resin wherein said resin has a total surface area of 40 to 200 square meters per gram in the range of pores above 30A° of which at least 30% of the pores have a radius of 30 to 250A° and a water swelling capacity of less than 10% by volume and wherein said system has a binding efficiency of at least 90% when an enzyme load of 500 U/ml carrier is offered or at least 80% when an enzyme load of 1000 U/ml carrier is offered, and an enzyme half-life during isomerization of at least 10 days.

In our own earlier European Patent Application 80.100449.0 (published under No. 14866) we have disclosed and claimed the use of an identical phenolic resin with the sole exception, that the resin was said to have a water swelling capacity of at least about 10% by volume. It was now discovered in accordance with this invention, that the water swelling capacity of the phenolic resin is not critical. The present invention therefore is directed to the use of phenolic resins of the specified kind without limitation in respect of the water swelling capacity, disclaiming at the same time those resins having a water swelling capacity of at least about 10% by volume, which are disclosed and claimed in the European Patent Application 80.100449.0 or, with other words, the present invention is concerned with the use of such resins having a water swelling capacity of less than 10% by volume.

Preferably the porous phenolic resin is an anion exchange type resin. Preferred resins are those, in which of the pores having a radius of about 30 to 250Å at least 50% have a radius of 50 to 150Å, and/or at least 20% have a radius of 60 to 90Å, and/or at least 20% have a radius of 70 to 80Å.

This invention is directed to a single use system which provides high binding capacity for glucose

2

# O 036 662

isomerase, high flow rates, long column lives and low enzyme consumption, thus making it extremely attractive for use in an economical commercial process.

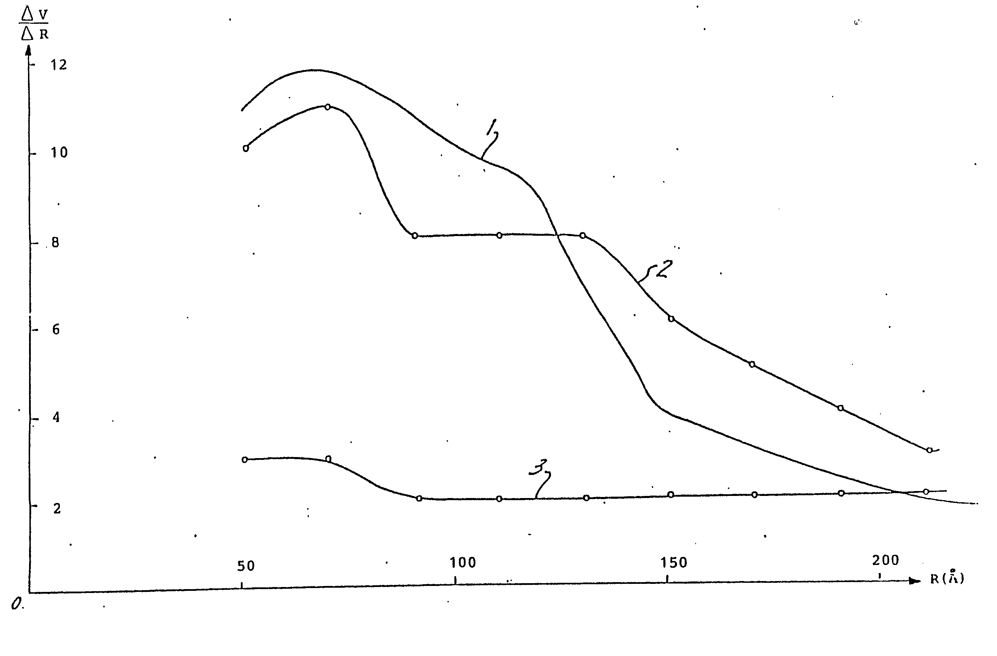

The Figure is a differential pore radius distribution curve of several carriers, showing the carrier of this invention as curve 1.

More in detail, the porous phenolic resin carriers defined in the claims have a porosity profile similar to curve 1 in the Figure. This curve was obtained by measuring pore size and volume by mercury porosimetry, plotting an integral curve expressing the relation between the volume condensed in the pores and the radii, and differentiating the integral curve to obtain the more illustrative differential distribution curve which expresses the increment of pore volume corresponding to a differential increase in their radius. A detailed description of this measurement, together with a description of the measurement of surface area, is set forth in "Adsorption On Solids", V. Ponec, et al., Butterworths, 1974, pages 38—39, 540—545 and 552—555.

While any phenolic resin having the surface area and porosity defined herein can be used in the practice of this invention, it is preferred to employ a resin having an anion exchange capacity. A specific resin found to be useful is Duolite ES—568[R]. This is a phenol-formaldehyde resin available from Diamond Shamrock.

The specific glucose isomerase enzyme employed in the practice of the invention is derived from *Streptomyces olivochromogenes* ATCC Nos. 21,114; 21,713; 21,714 or 21,715. Deposited strains ATCC Nos. 21,713; 21,714 and 21,715 are mutant strains of *Streptomyces olivochromogenes* ATCC No. 21,114. The glucose isomerase can also be derived from mutant strains of the ATCC No. 21,715 microorganism. As pointed out earlier in this specification, a commercially available glucose isomerase that is derived from the ATCC No. 21,715 microorganism or mutant strains thereof is sold under the name G—993 by CPC International Inc., International Plaza, Englewood Cliffs, New Jersey, U.S.A. The liquid enzyme concentrate is made in accordance with the procedure described in U.S. Patent No. 4,077,842. Regarding the activity of the enzyme, one unit (referred to as U) is defined as the amount of enzyme which produces one micromole of levulose per minute, in a 0.1 molar solution of glucose at pH 7.5 and 60°C in the presence of 0.01 molar $MgCl_2$ and 0.001 molar $CoCl_2$, using the following assay procedure, involving making a spectrophotometic determination of the ketose produced from a glucose solution under a standardized set of conditions.

A stock solution is made up in the following manner:

| Stock Solution for Assay | |
|---|---|
| Component | Amount |
| 0.01 M $MgCl_2$ | 1 ml |
| 0.001 M $CoCl_2$ | 1 ml |
| 1.0 M Phosphate buffer, pH 7.5 | 0.5 ml |
| Anhydrous D-glucose | 1.44 g. |
| Distilled water | To make up a total volume of 7.5 ml. |

The enzyme preparation to be assayed is first diluted to contain from 1 to 6 U/ml.

An enzymatic isomerization is conducted by adding 1 ml of the enzyme preparation to 3 ml of the stock solution, and incubating for 30 minutes at 60°C. At the end of the incubation period, a 1 ml aliquot is taken and quenched in a 9 ml volume of 0.5 N perchloric acid. The quenched aliquot is then diluted to a total volume of 250 ml. As a control, for comparative purposes, a glucose blank is also run by substituting 1 ml of water for 1 ml of the enzyme preparation in solution form, at the beginning of the incubation period. The ketose is then determined by a cysteine-sulfuric acid method. For the purposes of this assay, one U is defined as the amount of enzyme activity that is required to produce one micromole of levulose per minute under the isomerization conditions described above.

In order to prepare the immobilized system of this invention, the porous phenolic resin is mixed with glucose isomerase solution consisting of between about 100 and 1000 units of enzyme solution per ml of dry resin carrier. Preferably the enzyme preparation is very concentrated and contains from about 1000 to about 2500 Units per ml of enzyme solution. The porous resin is first washed, preferably with demineralized water.

3

Then, the resin is treated to adjust the pH to between 7.0 and 9.0, preferably between 7.5 and 8.5. It is preferred to employ a caustic wash step prior to the final pH adjustment in order to obtain a product pH of close to 8.2, thus optimizing the conversion to levulose. After the desired pH has been obtained, the enzyme adsorption step is carried out for at least 20 hours, depending on such factors as enzyme solution concentration and the like. A final wash may be carried out at this point to insure that the pH is still within the desired range. Once the enzyme is immobilized, the resulting system can be used immediately, or stored either in water or as a moist system until used.

In use, the system is reacted with a starch hydrolysate solution. The term "starch hydrolysate" is used to refer to a syrup or dry product that is made by acid and/or enzymatic hydrolysis of starch. A preferred type of starch hydrolysate for use for isomerization in accordance with the present invention is produced by acid or enzyme thinning to a D. E. of 20 or less followed by enzymatic saccharification to a D. E. above 90, preferably about 95. The term "D. E." is an abbreviation of "dextrose equivalent" and refers to the reducing sugar content of the material, calculated as dextrose and expressed as percent of total solids. During the saccharification process, the pH should be in the range between about 4.0 and about 5.0. Various buffers known to those skilled in the art may be used to achieve the desired pH.

In the preferred continuous process, the porous phenolic resin particles are placed in a plugged flow-through column and the enzyme immobilization procedure carried out prior to passing the starch hydrolysate through the column. Preferably the concentration of the starch hydrolysate solution is at least about 30% by weight. Flow rate, which is referred to as BVH, is adjusted during the process to assure maximum isomerization, e.g., about 45% conversion of the glucose to fructose. BVH is the column flow rate in bed volumes per hour.

In an alternative embodiment of the process of this invention, between about 50 to about 600 ppm of SO$_2$ is added to the starch hydrolysate supply. Preferably at least about 300 ppm SO$_2$ is added in order to enhance the stability of the glucose isomerase.

The following examples will serve to illustrate the practice of this invention:

Examples 1—6

In order to demonstrate that the system and process of this invention can be used on a plant scale, runs were made in columns 3 meters high and having a diameter of 50 cm. The previously mentioned glucose isomerase solution based on G—993 glucose isomerase was added to Duolite ES—568, which had been pre-treated with sodium hydroxide in a batch system.

Each of the columns was then packed with 600 liters of the resulting carrier-enzyme system. Then, the columns were fed with a 96 DE hydrolysate solution at 50% d.s. containing 150 ppm Mg++ as magnesium sulfate and adjusted with sodium hydroxide to a pH of 7.7—8.0. The initial column temperature is indicated in the table below. Since a relatively constant throughput was desired, the columns were operated by means of a compromise between a constant temperature and a variable temperature process, the respective end temperature being also indicated in the table below. The results, illustrative of plant scale runs, are given in the table below, but should not be understood to reflect the best results, which can be achieved with this invention.

TABLE

| EXAMPLES | | 1 | 2 | 3 | 4 | 5 | 6 |
|---|---|---|---|---|---|---|---|
| Enzyme Offered (U/ml Carrier) | | 367 | 364 | 375 | 508 | 435 | 377 |
| % Binding | | 99 | 99 | 98 | 94 | 96 | 98 |
| Initial BVH 45% | | 3.3 | 3.4 | 3.4 | 3.4 | 3.0 | 3.0 |
| Days of Operation | | 82 | 75 | 73 | 67 | 81 | 82 |
| Enzyme Consumption (u/g d.s.) | | 0.167 | 0.210 | 0.198 | 0.281 | 0.201 | 0.170 |
| Temperature | initial | 53 | 53 | 53 | 53 | 54 | 53 |
| | end | 63 | 65 | 65 | 63 | 65 | 64 |

**0 036 662**

Claims

1. An immobilized glucose isomerase system comprising glucose isomerase derived from *Streptomyces olivochromogenes* ATCC Nos. 21,114; 21,713; 21,714 or 21,715 or mutants thereof immobilized on a porous phenolic resin wherein said resin has a total surface area of 40 to 200 square meters per gram in the range of pores above 30Å of which at least 30% of the pores have a radius of 30 to 250Å and a water swelling capacity of less than 10% by volume and wherein said system has a binding efficiency of at least 90% when an enzyme load of 500 U/ml carrier is offered or at least 80% when an enzyme load of 1000 U/ml carrier is offered, and an enzyme half-life during isomerization of at least 10 days.

2. The system of claim 1 wherein said porous phenolic resin is an anion exchange type resin.

3. The system of claims 1 or 2 wherein at least 50% of the pores having a radius of 30 to 250Å have a radius of 50 to 150Å.

4. The system of one of claims 1—3 wherein at least 20% of the pores having a radius of 30 to 250Å have a radius of 60 to 90Å.

5. The system of one of claims 1—4 wherein at least 20% of the pores having a radius of 30 to 250Å have a radius of 70 to 80Å.

6. A process for the isomerization of glucose comprising contacting a glucose-containing solution with the immobilized glucose isomerase system of claim 1.

7. A process for the isomerization of glucose comprising contacting a glucose-containing solution with the immobilized glucose isomerase system of claim 2.

8. A process for the isomerization of glucose comprising contacting a glucose-containing solution with the immobilized glucose isomerase system of claim 3.

9. A process for the isomerization of glucose comprising contacting a glucose-containing solution with the immobilized glucose isomerase system of claim 4.

10. A process for the isomerization of glucose comprising contacting a glucose-containing solution with the immobilized glucose isomerase system of claim 5.

**Patentansprüche**

1. Immobilisiertes Glucoseisomerase-System aus Glucoseisomerase stammend von Streptomyces olivochromogenes ATCC Nr. 21,114; 21,713; 21,714 oder 21,715 oder Mutanten davon, immobilisiert auf einem porösen phenolischen Harz, wobei das Harz eine Gesamtoberfläche von 40 bis 200 m²/g im Bereich der Poren oberhalb 30Å, von welchen mindestens 30% der Poren einen Radius von 30 bis 250Å aufweisen und eine Wasserschwellkapazität von weniger als 10 Volumen-% besitzt und wobei dieses System eine Bindungswirksamkeit von mindestens 90% besitzt, wenn eine Enzymbelastung von 500 Einheiten/ml Träger angeboten wird oder von mindestens 80% wenn eine Enzymbelastung von 1000 Einheiten/ml Träger angeboten wird, und eine Enzym-Halbwertszeit während der Isomerisierung von mindestens 10 Tagen.

2. System gemäß Anspruch 1, worin das poröse Phenolharz ein Harz vom Anionenaustauscher-Typ ist.

3. System gemäß Ansprüchen 1 oder 2 worin mindestens 50% der Poren mit einem Radius von 30 bis 250Å einen Radius von 50 bit 150Å besitzen.

4. System gemäß einem der Ansprüche 1 bis 3, worin mindestens 20% der Poren mit einem Radius von 30 bis 250Å einen Radius von 60 bis 90Å besitzen.

5. System gemäß einem der Ansprüche 1 bis 4, worin mindestens 20% der Poren mit einem Radius von 30 bis 250Å einen Radius von 70 bis 80Å besitzen.

6. Verfahren zur Isomerisierung von Glucose, wobei eine Glucose-enthaltende Lösung mit einem immobilisierten Glucoseisomerase-System gemäß Anspruch 1 in Berührung gebracht wird.

7. Verfahren zur Isomerisierung von Glucose, wobei eine Glucose-enthaltende Lösung mit einem immobilisierten Glucoseisomerase-System gemäß Anspruch 2 in Berührung gebracht wird.

8. Verfahren zur Isomerisierung von Glucose, wobei eine Glucose-enthaltende Lösung mit einem immobilisierten Glucoseisomerase-System gemäß Anspruch 3 in Berührung gebracht wird.

9. Verfahren zur Isomerisierung von Glucose, wobei eine Glucose-enthaltende Lösung mit einem immobilisierten Glucoseisomerase-System gemäß Anspruch 4 in Berührung gebracht wird.

10. Verfahren zur Isomerisierung von Glucose, wobei eine Glucose-enthaltende Lösung mit einem immobilisierten Glucoseisomerase-System gemäß Anspruch 5 in Berührung gebracht wird.

**Revendications**

1. Système de glucose isomérase immobilisée comportant de la glucose isomérase dérivée des streptomyces olivochromogènes ATCC n°s 21.114, 21.713; 21.714 ou 21.715 ou des mutants de ceux-ci, immobilisée sur une résine phénolique poreuse, ladite résine présentant une surface totale de 40 à 200 m²/g dans la plage de pores·supérieure à 30 Å, dont au moins 30% des pores présentent un rayon de 30 à 250 Å, et une capacité de gonflement à l'eau inférieure à 10% en volume; et ledit système ayant une efficacité de liant d'au moins 90%, lorsqu'on présente une charge d'enzymes de 500 U/ml de support, ou d'au moins 80%, lorsqu'on présente une charge d'enzymes de 1000 U/ml de support, ainsi qu'une demi-vie d'enzyme d'au moins 10 jours pendant l'isomérisation.

2. Système de la revendication 1 dans lequel ladite résine phénolique poreuse est une résine du type résine échangeuse d'anions.

3. Système des revendications 1 ou 2 dans lequel au moins 50% des pores présentant un rayon de 30 à 250 Å, one un rayon de 50 à 150 Å.

4. Système de l'une des revendications 1 à 3 dans lequel au moins 20% des pores présentant un rayon de 30 à 250 Å, ont un rayon de 60 à 90 Å.

5. Système de l'une des revendications 1 à 4 dans lequel au moins 20% des pores présentant un rayon de 30 à 250 Å, ont un rayon de 70 à 80 Å.

6. Procédé d'isomérisation de glucose consistant à mettre en contact une solution contenant du glucose et le système de glucose isomérase immobilisée de la revendication 1.

7. Procédé d'isomérisation de glucose consistant à mettre en contact une solution contenant du glucose et le système de glucose isomérase immobilisée de la revendication 2.

8. Procédé d'isomérisation de glucose consistant à mettre en contact une solution contenant du glucose et le système de glucose isomérase immobilisée de la revendication 3.

9. Procédé d'isomérisation de glucose consistant à mettre en contact une solution contenant du glucose et le système de glucose isomérase immobilisée de la revendication 4.

10. Procédé d'isomérisation de glucose consistant à mettre en contact une solution contenant du glucose et le système de glucose isomérase immobilisée de la revendication 5.

0036662